# EUROPEAN PATENT APPLICATION

(11) **EP 4 249 922 A1**
(43) Date of publication of application: **27.09.2023**
(21) Application number: 23163151.6
(22) Date of filing: 21.03.2023
(51) Int. Cl.: G01N 35/10, G01N 33/49, G01N 15/05, G01N 15/00

(54) **BLOOD SAMPLE ANALYZER AND CONTROL METHOD THEREFOR**

(30) Priority: 21.03.2022 CN 202210279008
(71) Applicant: Shenzhen Mindray Bio-Medical Electronics Co., Ltd., Shenzhen, Guangdong 518057 (CN)
(72) Inventor: XIAO, Jiafan, Shenzhen, 518057 (CN); SHI, Huilin, Shenzhen, 518057 (CN); WANG, Fengming, Shenzhen, 518057 (CN)
(74) Representative: KIPA AB

(57) **Abstract**

The disclosure is applicable to the field of ESR test apparatuses, and discloses a blood sample analyzer and a method for controlling the blood sample analyzer. The blood sample analyzer includes an ESR test device, a sample collecting and dispensing device, a first cleaning liquid supply device, and an analysis and control device. The ESR test device includes an ESR test pipe and a photoelectric measurement component, the ESR test pipe being configured to provide a test place for erythrocyte aggregation of a blood sample, and the photoelectric measurement component being configured to measure a level of erythrocyte aggregation of the blood sample in the ESR test pipe. The analysis and control device is configured to analyze measurement data related to the level of erythrocyte aggregation measured by the photoelectric measurement component to obtain an erythrocyte sedimentation rate of the blood sample; and control delivery of an alkaline cleaning liquid supplied by the first cleaning liquid supply device to the ESR test pipe after completion of an ESR test in the ESR test pipe, so as to clean the ESR test pipe. The disclosure improves the accuracy and repeatability of ESR tests of blood samples.

## Description

### TECHNICAL FIELD

The disclosure relates to the field of ESR test apparatuses, and in particular to a blood sample analyzer and a method for controlling the blood sample analyzer.

### BACKGROUND

A blood sample analyzer provided in the related art integrates both a blood routine test function and an ESR test function. In an ESR test method, a photoelectric measurement component is used to measure a level of erythrocyte aggregation of a blood sample in an ESR test pipe, and a control device analyzes measurement data related to the level of erythrocyte aggregation measured by the photoelectric measurement component to obtain an erythrocyte sedimentation rate (ESR) of the blood sample. After completion of the ESR test in the ESR test, the ESR test pipe is cleaned with a same neutral cleaning liquid as that for a blood routine test device. The neutral cleaning liquid can remove contamination caused by particles, such as cells, in the previous blood sample remaining in pipes to prevent abnormal particle counts of the next blood sample due to the residues, so as to enable the cleaned liquid path components to meet cleaning requirements of the pipes after the blood routine test.

However, it was not found in the related art that, in the ESR test pipe that has been cleaned with a neutral cleaning liquid, there are still residues that affect the accuracy of the ESR test, which specifically lies in that there are residues of organic matters, such as proteins and cells, on pipes in contact with the blood sample after each test. The proteins, such as fibrinogen, in the blood sample remaining in the pipes have strong adhesion and are difficult to remove, but have little effect on the blood routine test. Thus, the blood routine test system can meet residual contamination requirements by means of cleaning the pipes with a neutral cleaning liquid after each test. However, in the related art mentioned above, in the ESR test method, the ESR is mainly reflected by the erythrocyte aggregation rate of rouleaux formation in a first stage during the ESR test. Positively charged proteins, such as fibrinogen and globulin, remaining in the ESR test pipe will increase the concentration of the positively charged proteins in the blood sample during the ESR test, resulting in acceleration of erythrocyte sedimentation; and albumin remaining in the ESR test pipe will increase the concentration of albumin in the blood sample during the ESR test, resulting in the decrease of ESR. As a result, the proteins in the blood sample remaining in the pipe will cause deviation of an ESR test value of the subsequent blood sample and thus affect the accuracy of the ESR test result.

It should be noted herein that the statement in the background section merely provides the background related to the disclosure and does not necessarily constitute the prior art.

### SUMMARY

A first objective of the disclosure is to provide a blood sample analyzer, aiming to solve the technical problem in the related art of low accuracy of the ESR test results caused by cleaning of an ESR test pipe with a neutral cleaning liquid.

In order to achieve the above objective, the disclosure provides the following solution: a blood sample analyzer, including:
an ESR test device including an ESR test pipe and a photoelectric measurement component, the ESR test pipe being configured to provide a test place for erythrocyte aggregation of a blood sample, and the photoelectric measurement component being configured to measure a level of erythrocyte aggregation of the blood sample in the ESR test pipe;
a sample collecting and dispensing device configured to aspirate the blood sample from a sample container and dispense at least part of the aspirated blood sample to the ESR test pipe;
a first cleaning liquid supply device configured to supply an alkaline cleaning liquid; and
an analysis and control device configured to:
   analyze measurement data related to the level of erythrocyte aggregation measured by the photoelectric measurement component to obtain an erythrocyte sedimentation rate of the blood sample; and
   control delivery of the alkaline cleaning liquid supplied by the first cleaning liquid supply device to the ESR test pipe after completion of an ESR test in the ESR test pipe so as to clean the ESR test pipe.

A second objective of the disclosure is to provide a method for controlling a blood sample analyzer, including:
controlling a sample collecting and dispensing device to aspirate a blood sample from a sample container;
controlling the sample collecting and dispensing device to dispense at least part of the aspirated blood sample to an ESR test pipe;
controlling a photoelectric measurement component to measure a level of erythrocyte aggregation of the blood sample in the ESR test pipe;
analyzing measurement data related to the level of erythrocyte aggregation measured by the photoelectric measurement component to obtain an erythrocyte sedimentation rate of the blood sample; and
controlling delivery of an alkaline cleaning liquid supplied by a first cleaning liquid supply device to the ESR test pipe, so as to clean the ESR test pipe.

According to the blood sample analyzer and the control method therefor provided in the disclosure, measuring the erythrocyte sedimentation rate of a blood sample in a test pipe using an erythrocyte aggregation method (also referred to as pipe-based dynamic spectrophotometry) can greatly improve the efficiency of the ESR test as compared with the solution of measuring the erythrocyte sedimentation rate of a blood sample in a test tube using the Westergren method. In addition, according to the disclosure, the blood sample analyzer is provided with a first cleaning liquid supply device that can supply an alkaline cleaning liquid, and the alkaline cleaning liquid supplied by the first cleaning liquid supply device is delivered to the ESR test pipe after completion of an ESR test in the ESR test pipe, so as to clean the ESR test pipe, which can effectively remove protein residues in the ESR test pipe and thus improve the accuracy and repeatability of ESR tests of subsequent blood samples.

### BRIEF DESCRIPTION OF THE DRAWINGS

FIG. 1 is a schematic diagram showing a structure of a blood sample analyzer according to a first embodiment of the disclosure;
FIG. 2 is a schematic diagram showing a pipe connection of an ESR test pipe to a sample collecting and dispensing device and a first cleaning liquid supply device according to the first embodiment of the disclosure;
FIG. 3 is a schematic diagram showing a state of the blood sample analyzer according to the first embodiment of the disclosure when a sample collecting needle moves to a position above a sample container;
FIG. 4 is a schematic diagram showing a pipe connection of an ESR test pipe to a sample collecting and dispensing device, a first cleaning liquid supply device and a waste liquid cell according to a second embodiment of the disclosure;
FIG. 5 is a schematic diagram showing a pipe connection of an ESR test pipe to a sample collecting and dispensing device, a first cleaning liquid supply device and a waste liquid cell according to a third embodiment of the disclosure;
FIG. 6 is a schematic diagram showing a pipe connection of an ESR test pipe to a sample collecting and dispensing device and a first cleaning liquid supply device according to a fourth embodiment of the disclosure;
FIG. 7 is a schematic diagram showing a pipe connection of an ESR test pipe to a sample collecting and dispensing device and a first cleaning liquid supply device according to a fifth embodiment of the disclosure;
FIG. 8 is a schematic diagram showing a pipe connection of an ESR test pipe to a sample collecting and dispensing device and a first cleaning liquid supply device according to a sixth embodiment of the disclosure;
FIG. 9 is a schematic diagram showing a pipe connection of an ESR test pipe to a sample collecting and dispensing device and a first cleaning liquid supply device according to a seventh embodiment of the disclosure; and
FIG. 10 is a schematic diagram showing a pipe connection of an ESR test pipe to a sample collecting and dispensing device and a first cleaning liquid supply device according to an eighth embodiment of the disclosure.

### List of reference numerals:

10 blood sample analyzer; 100 ESR test device; 110 ESR test pipe; 120 photoelectric measurement component; 121 light-emitting diode; 122 photodiode; 130 heater; 140 temperature sensor; 200 sample collecting and dispensing device; 210 sample collecting needle; 220 sample aspiration pipe; 230 first power source; 240 fourth control valve; 250 movement driving device; 251 transverse guide rail; 252 transverse movement component; 253 vertical guide rail; 254 vertical movement component; 260 third power source; 300 first cleaning liquid supply device; 310 first liquid storage container; 320 second power source; 330 fifth control valve; 400 analysis and control device; 500 second cleaning liquid supply device; 600 blood routine test device; 610 test cell; 700 cleaning cell; 800 temporary storage cell; 101 first control valve; 102 waste liquid cell; 103 first liquid discharge pipe; 104 sixth control valve; 105 seventh control valve; 106 eighth control valve; 107 second control valve; 108 third control valve; 109 ninth control valve; 20 sample container.

### DESCRIPTION OF THE PREFERRED EMBODIMENTS

The technical solutions in the embodiments of the disclosure will be described below clearly and completely in conjunction with the accompanying drawings in the embodiments of the disclosure. Obviously, the embodiments described are merely some rather than all of the embodiments of the disclosure.

### First embodiment:

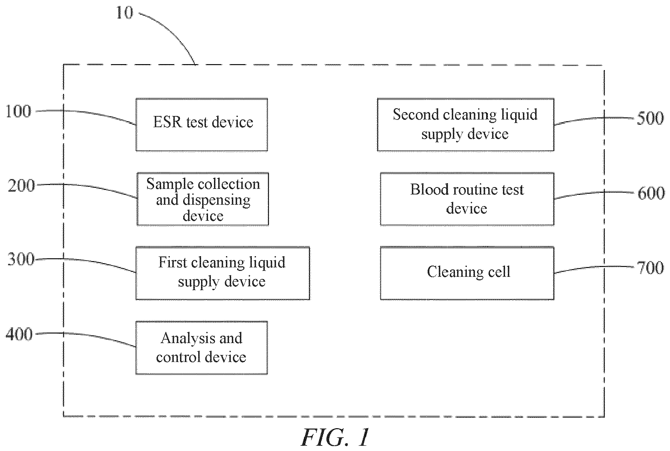

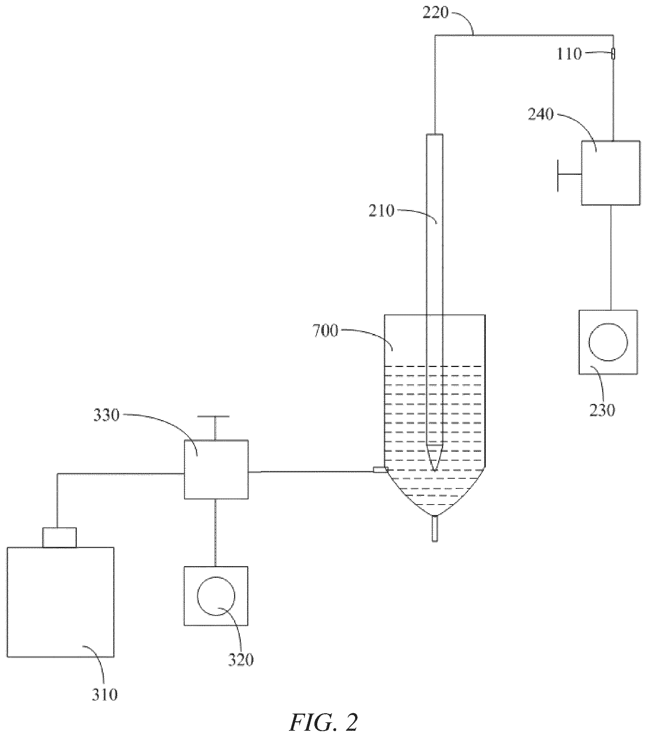

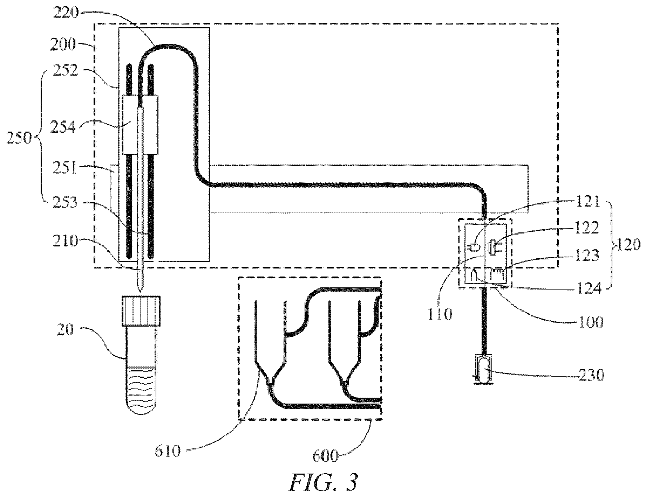

As shown in FIGS. 1 to 3, a blood sample analyzer 10 according to the first embodiment of the disclosure includes an ESR test device 100, a sample collection and dispensing device 200, a first cleaning liquid supply device 300, and an analysis and control device 400. The sample collection and dispensing device 200 is configured to aspirate a blood sample from a sample container 20 and dispense at least part of the aspirated blood sample to the ESR test device 100. The ESR test device 100 is configured to receive the blood sample dispensed by the sample collection and dispensing device 200 and perform an ESR test on the dispensed blood sample. The first cleaning liquid supply device 300 is configured to clean the ESR test device 100 after completion of an ESR test. The analysis and control device 400 is configured to analyze the test data obtained by the ESR test device 100 to obtain the erythrocyte sedimentation rate of the blood sample, and is configured to control delivery of a cleaning liquid supplied by the first cleaning liquid supply device 300 to the ESR test device 100 after completion of the ESR test in the ESR test device 100 to clean the ESR test device 100.

As an implementation, the ESR test device 100 includes an ESR test pipe 110 and a photoelectric measurement component 120, the ESR test pipe 110 being configured to provide a test place for erythrocyte aggregation of the blood sample, and the photoelectric measurement component 120 being configured to measure a level of erythrocyte aggregation of the blood sample in the ESR test pipe 110. The sample collecting and dispensing device 200 is configured to aspirate the blood sample from the sample container 20 and dispense at least part of the aspirated blood sample to the ESR test pipe 110. The analysis and control device 400 is configured to process measurement data related to the level of erythrocyte aggregation measured by the photoelectric measurement component 120 to obtain an erythrocyte sedimentation rate of the blood sample. In this embodiment, the erythrocyte sedimentation rate of the blood sample in the pipe is measured using an erythrocyte aggregation method (also referred to as pipe-based dynamic spectrophotometry), that is, an erythrocyte sedimentation distance is predicted by measuring the level of erythrocyte aggregation within a specified period of time. The blood sample is a whole blood sample, and erythrocytes in the whole blood sample aggregate from a dispersed state to form a rouleaux structure before sedimentation begins, and then aggregate into cluster, and thus the sedimentation begins, wherein the rate of sedimentation thereof mainly depends on the level of aggregation. A change in the erythrocyte aggregation state will lead to a change in the scattering effect of the erythrocytes on a measurement light beam, which specifically lies in that the light transmittance thereof increases with the increase of the level of aggregation. Thus, the light transmittance of the whole blood sample over time can be measured to obtain the level of erythrocyte aggregation and then obtain the erythrocyte sedimentation rate.

As an implementation, the first cleaning liquid supply device 300 is configured to supply an alkaline cleaning liquid, specifically a cleaning liquid with an alkaline pH (i.e., pH is greater than 7). The analysis and control device 400 is further configured to control delivery of the alkaline cleaning liquid supplied by the first cleaning liquid supply device 300 to the ESR test pipe 110 after completion of the ESR test in the ESR test pipe 110, so as to clean the ESR test pipe 110. After completion of the ESR test in the ESR test pipe 110, there will be protein residues on the ESR test pipe 110. If the protein residues on the ESR test pipe 110 are not thoroughly removed before the erythrocyte sedimentation test, the concentration of positively charged proteins, such as fibrinogen and globulin, in the blood sample will increase, resulting in acceleration of erythrocyte sedimentation, and the concentration of albumin will increase to hinder acceleration of erythrocyte sedimentation. Thus, the proteins in the blood sample remaining in the pipe will cause deviation of ESR test values of subsequent blood samples. In this embodiment, cleaning the ESR test pipe 110 with the alkaline cleaning liquid can effectively remove the proteins remaining in the ESR test pipe 110 and thus improve the accuracy and repeatability of ESR tests of subsequent blood samples.

As an implementation, the photoelectric measurement component 120 includes a light-emitting diode 121 and a photodiode 122, which are arranged on two sides of the ESR test pipe 110. Specifically, at the beginning of the ESR test, the erythrocytes are in a monodisperse state, the blood sample has the lowest light transmittance, and the photodiode 122 has the lowest output value of Uo; and as the erythrocytes aggregate into a rouleaux structure, the light transmittance of the blood sample increases, and the output value of the photodiode 122 increases to Ui. The level of erythrocyte aggregation is directly proportional to the output change Ui-Uo of the photodiode 122, and the erythrocyte sedimentation rate, i.e., ESR, is then obtained by look-up table.

As an implementation, the ESR test device 100 further includes a heater 130 and a temperature sensor 140. The heater 130 is configured to heat the blood sample in the ESR test pipe 110, and the temperature sensor 140 is configured to measure and back-feed the temperature. In this way, the heater 130 cooperates with the temperature sensor 140 to achieve the temperature control of the ESR test device 100 and the ESR test pipe 110 passing through the inside of the ESR test device, and the light-emitting diode 121 cooperates with the photodiode 122 to achieve the measurement of light absorption or light scattering of the blood sample in the ESR test pipe 110, such that the whole ESR test process can be completed within one minute, thereby effectively improving the efficiency of the ESR test.

As an implementation, the ESR test pipe 110 is a capillary tube, such that the blood consumption for the ESR test can be small, the ESR test pipe 110 can have small compressive deformation so as to achieve more accurate quantitative measurement, and it is also possible to heat the ESR test pipe 110 more uniformly and arrange the ESR test pipe 110 in a convenient layout.

Specifically, the alkaline cleaning liquid is a cleaning liquid that can remove the protein residues in the ESR test pipe 110 and has an alkaline pH value (i.e., pH is greater than 7).

As an implementation, the alkaline cleaning liquid contains at least one surfactant, which is conducive to ensuring the residue removal ability of the alkaline cleaning liquid.

As an implementation, the blood sample analyzer 10 further includes a second cleaning liquid supply device 500. The second cleaning liquid supply device 500 is configured to supply a neutral cleaning liquid. The neutral cleaning liquid is specifically a cleaning liquid with a neutral pH value (i.e., pH is equal to 7). The analysis and control device 400 is further configured to deliver, after completion of the ESR test in the ESR test pipe 110, the alkaline cleaning liquid supplied by the first cleaning liquid supply device 300 to the ESR test pipe 110 to clean the ESR test pipe 110, and then deliver the neutral cleaning liquid supplied by the second cleaning liquid supply device 500 to the ESR test pipe 110 to clean the ESR test pipe 110.

Specifically, after completion of one ESR test, the ESR test pipe 110 is sequentially cleaned with the alkaline cleaning liquid and cleaned with the neutral cleaning liquid, so as to ensure that the ESR test pipe 110 does not affect measurement of a level of erythrocyte aggregation of a next blood sample.

As an implementation, the blood sample analyzer 10 further includes a blood routine test device 600. The blood routine test device 600 includes a test cell 610 and a blood routine test component. The sample collecting and dispensing device 200 is further configured to dispense at least part of the aspirated blood sample to the test cell 610 or to a reaction cell that is connected to the test cell 610 by means of a pipe. The blood routine test component is configured to perform a blood routine test on the blood sample in the test cell 610, the blood routine test including at least one of differential leukocyte count, reticulocyte count, hemoglobin measurement, erythrocyte count and platelet count. During the differential leukocyte count and the reticulocyte count, the sample collecting and dispensing device 200 dispenses at least part of the aspirated blood sample to the reaction cell, and then delivers a test sample, which is prepared from the blood sample and a reagent in the reaction cell, to the test cell (also referred to as a flow cell) for differential leukocyte count or reticulocyte count by means of a pipe. During the hemoglobin measurement, the sample collecting and dispensing device 200 dispenses at least part of the aspirated blood sample to the test cell for hemoglobin measurement. During the erythrocyte count and the platelet count, when a sheath flow impedance method is used, the sample collecting and dispensing device 200 dispenses the at least part of the aspirated blood sample to the test cell for hemoglobin measurement, and then delivers a test sample, which is prepared from the blood sample and a reagent in the test cell for hemoglobin measurement, to a sheath flow impedance cell by means of a pipe; and when a non-impedance method is used, the sample collecting and dispensing device 200 directly dispenses the at least part of the aspirated blood sample to the test cell for erythrocyte count and platelet count.

As an implementation, the analysis and control device 400 is further configured to control delivery of the neutral cleaning liquid supplied by the second cleaning liquid supply device 500 to the test cell 610 after completion of the blood routine test in the test cell 610, so as to clean the test cell 610. In this embodiment, the neutral cleaning liquid for cleaning the test cell 610 and the neutral cleaning liquid for cleaning the ESR test pipe 110 are both supplied by the same cleaning liquid supply device, which is conductive to reducing the size and cost of the blood sample analyzer 10. When the blood routine test device 600 further includes a reaction cell, the neutral cleaning liquid supplied by the second cleaning liquid supply device 500 may be configured to clean the reaction cell and the pipe that is connected between the test cell 610 and the reaction cell. Certainly, in the specific application, as an alternative embodiment, the neutral cleaning liquid for cleaning the test cell 610 and the neutral cleaning liquid for cleaning the ESR test pipe 110 may also be respectively supplied by two different cleaning liquid supply devices.

As an implementation, the sample collecting and dispensing device 200 includes a sample collecting needle 210, a sample aspiration pipe 220 and a first power source 230. The sample aspiration pipe 220 is connected to the sample collecting needle 210 and the first power source 230 respectively. The first power source 230 is configured to drive the sample collecting needle 210 to aspirate fluid and discharge fluid.

As an implementation, the ESR test pipe 110 is connected to the sample aspiration pipe 220, that is, the ESR test pipe 110 and the sample aspiration pipe 220 are two different pipes connected together. In this way, it is possible to only use a capillary tube with good transmission of light as the ESR test pipe 110, which can reduce the requirement on the sample aspiration pipe 220 and thus reduce the cost of the pipe. In this embodiment, the analysis and control device 400 is further configured to control the first power source 230 to drive the sample collecting needle 210 to aspirate the blood sample from the sample container 20, and control the first power source 230 to drive the sample collecting needle 210 to deliver at least part of the aspirated blood sample to the ESR test pipe 110. Certainly, in the specific application, as an alternative embodiment, the ESR test pipe 110 and the sample aspiration pipe 220 may also be integrated, that is, a portion of the sample aspiration pipe 220 is used as the ESR test pipe 110.

As an implementation, the analysis and control device 400 is configured to control the first power source 230 to dispense at least part of the blood sample aspirated by the sample collecting needle 210 to the test cell 610 or the reaction cell via the sample collecting needle 210, and then control the first power source 230 to deliver at least part of the blood sample aspirated by the sample collecting needle 210 to the ESR test pipe 110. In this embodiment, the sample is dispensed to the blood routine test device 600, and then the sample is dispensed to the ESR test device 100. Certainly, in the specific application, as an alternative embodiment, the sample may be dispensed to the ESR test device 100, and then the sample is dispensed to the blood routine test device 600, that is, the analysis and control device 400 may also be configured to control the first power source 230 to deliver at least part of the blood sample aspirated by the sample collecting needle 210 to the ESR test pipe 110, and then control the first power source 230 to dispense at least part of the blood sample aspirated by the sample collecting needle 210 to the test cell 610 or the reaction cell via the sample collecting needle 210.

As an implementation, the blood sample analyzer 10 further includes a cleaning cell 700, and the first cleaning liquid supply device 300 includes a first liquid storage container 310 and a second power source 320, the first liquid storage container 310 being configured to store the alkaline cleaning liquid, and the second power source 320 being connected to the first liquid storage container 310 and the cleaning cell 700 respectively. The analysis and control device 400 is further configured to control the second power source 320 to control delivery of the alkaline cleaning liquid in the first liquid storage container 310 to the cleaning cell 700; and control, after completion of the ESR test in the ESR test pipe 110, the first power source 230 to drive the sample collecting needle 210 to aspirate the alkaline cleaning liquid from the cleaning cell 700 and deliver the alkaline cleaning liquid to the ESR test pipe 110, so as to clean the ESR test pipe 110. The cleaning cell 700 is configured to store the alkaline cleaning liquid for each cleaning. The second power source 320 is configured to provide power for delivery of the alkaline cleaning liquid from the first liquid storage container 310 to the cleaning cell 700. The waste liquid formed after cleaning of the ESR test pipe 110 may be discharged to the cleaning cell 700 via the sample aspiration pipe 220 and the sample collecting needle 210, and is then discharged from the cleaning cell 700 to a waste liquid cell; or the waste liquid formed after cleaning of the ESR test pipe 110 may be directly discharged to a waste liquid cell via the sample aspiration pipe 220 and the sample collecting needle 210.

As an implementation, the cleaning cell 700 is provided with a first opening, a first port and a second port. The first opening is configured for the sample collecting needle 210 to be inserted into the cleaning cell 700 or pulled out of the cleaning cell 700 such that the sample collecting needle 210 can aspirate the alkaline cleaning liquid from the cleaning cell 700. The first port is configured to connect the first liquid storage container 310 and the second power source 320 by means of a pipe. The second port is configured to empty the cleaning cell 700, and may be connected to a waste liquid cell by means of a pipe.

As an implementation, the first power source 230 is a syringe. Certainly, in the specific application, the first power source 230 is arranged in a manner not limited thereto. For example, as an alternative embodiment, the second power source 320 is a liquid pump.

As an implementation, the first power source 230 is connected to the sample aspiration pipe 220 by means of a fourth control valve 240, and the fourth control valve 240 is a two-way valve for controlling connection and disconnection of the pipe. Certainly, in the specific application, as an alternative embodiment, the fourth control valve 240 may not be provided.

As an implementation, the second power source 320 is a syringe, and the second power source 320 is switchable to be connected to the first liquid storage container 310 and the cleaning cell 700 by means of a fifth control valve 330, and the fifth control valve 330 may be a three-way valve. Certainly, in the specific application, the second power source 320 and the fifth control valve 330 are arranged in a manner not limited thereto. For example, as an alternative embodiment, the fifth control valve 330 may also include two two-way valves; or, as another alternative embodiment, the second power source 320 is a liquid pump, and the fifth control valve 330 is a two-way valve.

As an implementation, the sample collecting and dispensing device 200 further includes a movement driving device 250 for driving the sample collecting needle 210 to move. The movement driving device 250 is connected to the sample collecting needle 210, and is configured to drive the sample collecting needle 210 to move in a predetermined direction in a spatial dimension, so as to move the sample collecting needle 210 to different working positions, such as a standby position, a sample collecting position, a sample dispensing position, etc.

As an implementation, the movement driving device 250 includes a transverse guide rail 251, a transverse movement component 252, a transverse driving component, a vertical guide rail 253, a vertical movement component 254 and a vertical driving component. The sample collecting needle 210 is mounted on the vertical movement component 254, the vertical guide rail 253 is mounted on the transverse movement component 252 or is integrally formed on the transverse movement component 252, the vertical movement component 254 is mounted on the vertical guide rail 253 in a vertically slidable manner, the vertical driving component is mounted on the transverse movement component 252 and connected to the vertical movement component 254, and the vertical driving component is configured to drive the vertical movement component 254 to move the sample collecting needle 210 up and down. The transverse movement component 252 is mounted on the transverse guide rail 251 in a transversely slidable manner, and the transverse driving component is connected to the transverse movement component 252 for driving the transverse movement component 252 to transversely move the vertical guide rail 253, the vertical movement component 254, the vertical driving component and the sample collecting needle 210 together. In the specific application, the transverse movement component 252 may drive the sample collecting needle 210 to slide to a position above the sample container 20 holding the blood sample or to a position above the test cell 610 or the reaction cell of the blood routine test device 600, or may drive the sample collecting needle 210 to move away from the position above the sample container 20 holding the blood sample or the position above the test cell 610 or the reaction cell of the blood routine test device 600. The vertical movement component 254 may drive the sample collecting needle 210 to descend to extend into the sample container 20, so as to aspirate the blood sample, and after completion of the sample aspiration, the vertical movement component 254 may also drive the sample collecting needle 210 to rise to the position above the sample container 20; and the vertical movement component 254 may also drive the sample collecting needle 210 to descend to a position suitable for dispensing the blood sample to the test cell 610 or the reaction cell of the blood routine test device 600, and after completion of the sample dispensing, the vertical movement component 254 may then drive the sample collecting needle 210 to rise to the position above the sample dispensing position. Certainly, in the specific application, the movement driving device 250 is arranged in a manner not limited thereto. For example, as an alternative embodiment, the sample collecting needle 210 may be mounted on the transverse movement component 252, and the transverse guide rail 251 and the transverse driving device may be provided on the vertical movement component 254; or, as another alternative embodiment, in addition to including the transverse guide rail 251, the transverse movement component 252, the transverse driving component, the vertical guide rail 253, the vertical movement component 254 and the vertical driving component, the movement driving device 250 may further include a longitudinal guide rail, a longitudinal movement component and a longitudinal driving component, the longitudinal guide rail and the transverse guide rail 251 being in two horizontal directions perpendicular to each other, that is, the sample collecting needle 210 may move in a three-dimensional directions; or, as yet another alternative embodiment, the movement driving device 250 may use a combination of a swing arm rotation mechanism and a lifting mechanism.

A method for controlling the blood sample analyzer 10 according to this embodiment includes:
controlling the sample collecting and dispensing device 200 to aspirate a blood sample from the sample container 20;
controlling the sample collecting and dispensing device 200 to dispense at least part of the aspirated blood sample to the ESR test pipe 110;
controlling the photoelectric measurement component 120 to measure a level of erythrocyte aggregation of the blood sample in the ESR test pipe 110;
analyzing measurement data related to the level of erythrocyte aggregation measured by the photoelectric measurement component 120 to obtain the erythrocyte sedimentation rate of the blood sample; and
controlling delivery of an alkaline cleaning liquid supplied by the first cleaning liquid supply device 300 to the ESR test pipe 110, so as to clean the ESR test pipe 110.

As an implementation, controlling the sample collecting and dispensing device 200 to aspirate a blood sample from the sample container 20 includes: controlling the first power source 230 to drive the sample collecting needle 210 to aspirate the blood sample from the sample container 20. Controlling the sample collecting and dispensing device 200 to dispense at least part of the aspirated blood sample to the ESR test pipe 110 includes: controlling the first power source 230 to drive the sample collecting needle 210 to deliver the at least part of the aspirated blood sample to the ESR test pipe 110.

As an implementation, controlling delivery of an alkaline cleaning liquid supplied by the first cleaning liquid supply device 300 to the ESR test pipe 110 includes: controlling the second power source 320 to drive delivery of the alkaline cleaning liquid in the first liquid storage container 310 to the cleaning cell 700; and controlling the first power source 230 to drive the sample collecting needle 210 to aspirate the alkaline cleaning liquid from the cleaning cell 700 and deliver the alkaline cleaning liquid to the ESR test pipe 110.

As an implementation, the control method further includes, after the ESR test pipe 110 is cleaned with the alkaline cleaning liquid, controlling delivery of the neutral cleaning liquid supplied by the second cleaning liquid supply device 500 to the ESR test pipe 110 to clean the ESR test pipe 110.

As an implementation, the control method further includes, before or after the sample collecting and dispensing device 200 is controlled to dispense the aspirated blood sample to the ESR test pipe 110,
controlling the sample collecting and dispensing device 200 to dispense at least part of the aspirated blood sample to the test cell 610 or the reaction cell; and
controlling the blood routine test component to perform a blood routine test on the blood sample in the test cell 610, the blood routine test including at least one of differential leukocyte count, reticulocyte count, hemoglobin measurement, erythrocyte count and platelet count;
analyzing and processing test data of the blood routine test component to obtain blood routine test parameters of the blood sample; and
controlling delivery of the neutral cleaning liquid supplied by the second cleaning liquid supply device 500 to the test cell 610, so as to clean the test cell 610.

In this embodiment, the process of cleaning the ESR test pipe 110 after the ESR test is as follows. The second power source 320 delivers a certain amount of the alkaline cleaning liquid to the cleaning cell 700, the first power source 230 drives the sample collecting needle 210 to aspirate the alkaline cleaning liquid from the cleaning cell 700 to the ESR test pipe 110, the blood sample remaining in the ESR test pipe 110 and the sample aspiration pipe 220 can be removed from wall surfaces to the alkaline cleaning liquid, the first power source 230 is then controlled to drive discharge of the cleaning liquid after cleaning of the ESR test pipe 110 to the cleaning cell 700 via the sample collecting needle 210, and finally the cleaning cell 700 is emptied. After cleaning the ESR test pipe 110 with the alkaline cleaning liquid, the ESR test pipe 110 is cleaned with the neutral cleaning liquid. After completion of one ESR test, the ESR test pipe 110 is sequentially cleaned with the alkaline cleaning liquid and the neutral cleaning liquid, so as to ensure that the ESR test pipe 110 does not affect the measurement of a level of erythrocyte aggregation of a next blood sample and also ensure the accuracy and repeatability of the ESR test on the premise of ensuring the efficiency of the ESR test.

### Second Embodiment:

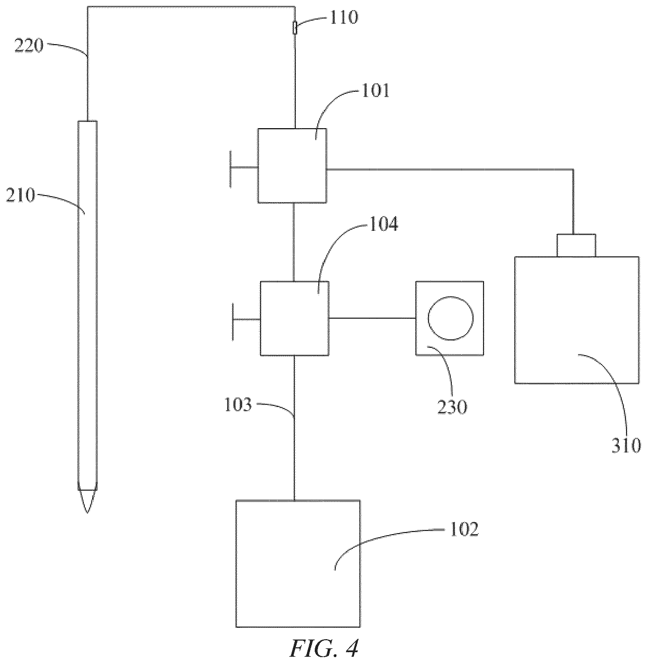

Referring to FIGS. 1 to 4, the blood sample analyzer 10 and the control method therefor according to this embodiment are different from those in the first embodiment mainly in the manner of driving the delivery of the alkaline cleaning liquid, which specifically lies in that, in the first embodiment, the delivery of the blood sample and the delivery of the alkaline cleaning liquid are driven by two mutually independent power sources, one of which is configured to deliver the alkaline cleaning liquid to the cleaning cell 700, and the other is configured to collect a sample, dispense the sample, and aspirate the alkaline cleaning liquid from the cleaning cell 700 to the ESR test pipe 110; while in this embodiment, the delivery of the blood sample and the delivery of the alkaline cleaning liquid are driven by a same power source, and no cleaning cell 700 is provided in this embodiment.

Specifically, in this embodiment, the first cleaning liquid supply device 300 includes a first liquid storage container 310. The first liquid storage container 310 is configured to store the alkaline cleaning liquid. The blood sample analyzer 10 further includes a first control valve 101, and the first power source 230 is switchable to be connected to the sample aspiration pipe 220 and the first liquid storage container 310 by means of the first control valve 101. The analysis and control device 400 is further configured to control, after completion of the ESR test in the ESR test pipe 110, the first control valve 101 to switch to a state in which the first power source 230 is in conduction with the first liquid storage container 310, then control the first power source 230 to aspirate the alkaline cleaning liquid from the first liquid storage container 310, then control the first control valve 101 to switch to a state in which the first power source 230 is in conduction with the sample aspiration pipe 220, and control the first power source 230 to deliver the aspirated alkaline cleaning liquid to the ESR test pipe 110, so as to clean the ESR test pipe 110. The blood sample and the alkaline cleaning liquid are delivered by the same power source, which can reduce the number of power sources of the blood sample analyzer 10 and is thus conductive to reducing the cost of the blood sample analyzer 10 and reducing the size of the blood sample analyzer.

As an implementation, the blood sample analyzer 10 further includes a waste liquid cell and a first liquid discharge pipe, the first liquid discharge pipe being connected between the waste liquid cell and the first power source 230. The analysis and control device 400 is further configured to control the first power source 230 to drive discharge of the cleaning liquid after cleaning of the ESR test pipe 110 to the waste liquid cell via the first liquid discharge pipe. In this embodiment, a front end of the ESR test pipe 110 is connected to the sample collecting needle 210, and a rear end of the ESR test pipe 110 is connected to the first liquid storage container 310, the first power source 230 and the first liquid discharge pipe by means of the first control valve 101 respectively.

As an implementation, the blood sample analyzer 10 further includes a sixth control valve 104, and the first power source 230 is switchable to be connected to the first liquid discharge pipe and the first control valve 101 by means of the sixth control valve 104.

Different from the first embodiment, in the method for controlling the blood sample analyzer 10 according to this embodiment, controlling delivery of the alkaline cleaning liquid supplied by the first cleaning liquid supply device 300 to the ESR test pipe 110 includes: controlling the first control valve 101 to switch to a state in which the first power source 230 is in conduction with the first liquid storage container 310, and controlling the first power source 230 to aspirate the alkaline cleaning liquid from the first liquid storage container 310; and controlling the first control valve 101 to switch to a state in which the first power source 230 is in conduction with the sample aspiration pipe 220, and controlling the first power source 230 to deliver the aspirated alkaline cleaning liquid to the ESR test pipe 110.

In this embodiment, the process of cleaning the ESR test pipe 110 after the ESR test is as follows. The first power source 230 delivers a certain amount of alkaline cleaning liquid to the ESR test pipe 110 and the sample aspiration pipe 220 by means of the first control valve 101, the blood sample remaining in the ESR test pipe 110 and the sample aspiration pipe 220 can be removed from the wall surfaces to the alkaline cleaning liquid, and the first power source 230 is then controlled to drive delivery of the cleaning liquid after cleaning of the ESR test pipe 110 to the waste liquid cell via the first liquid discharge pipe. After cleaning the ESR test pipe 110 with the alkaline cleaning liquid, the ESR test pipe 110 is cleaned with the neutral cleaning liquid.

In addition to the differences described above, other content of the blood sample analyzer 10 and the control method therefor according to this embodiment may be optimally designed with reference to the first embodiment, which will not be described in detail herein.

### Third Embodiment:

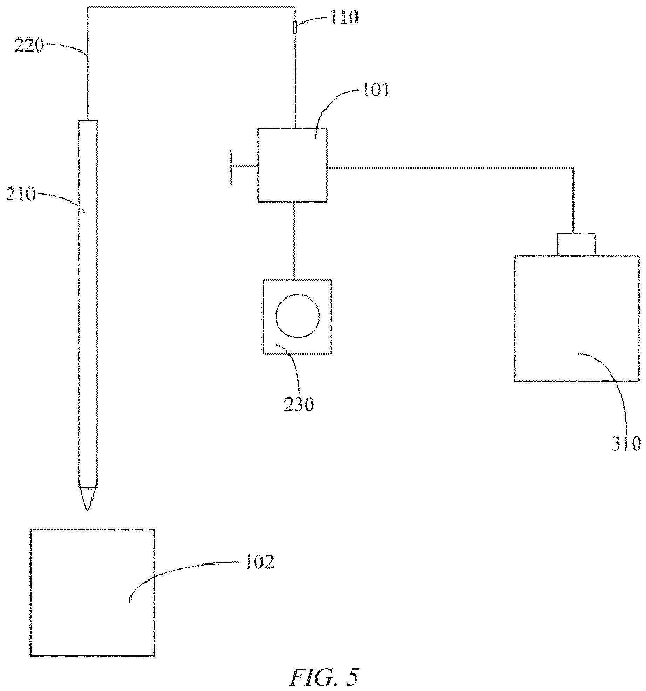

Referring to FIGS. 1, 4 and 5, the blood sample analyzer 10 and the control method therefor according to this embodiment are different from those in the second embodiment mainly in the manner of discharging the waste liquid after cleaning of the ESR test pipe 110, which specifically lies in that, in the second embodiment, the waste liquid after cleaning of the ESR test pipe 110 is discharged from the first liquid discharge pipe that is connected to the rear end of the ESR test pipe 110; while in this embodiment, the waste liquid after cleaning of the ESR test pipe 110 is discharged from the sample collecting needle 210 that is connected to the front end of the ESR test pipe 110.

Specifically, in this embodiment, the blood sample analyzer 10 also includes a waste liquid cell that is arranged separately from the first power source 230, that is, the waste liquid cell is not connected to the first power source 230 by means of a pipe. The analysis and control device 400 is further configured to control the first power source 230 to drive discharge of the cleaning liquid after cleaning of the ESR test pipe 110 to the waste liquid cell via the sample aspiration pipe 220 and the sample collecting needle 210.

In addition to the differences described above, other content of the blood sample analyzer 10 and the control method therefor according to this embodiment may be optimally designed with reference to the first embodiment and the second embodiment, which will not be described in detail herein.

### Forth Embodiment:

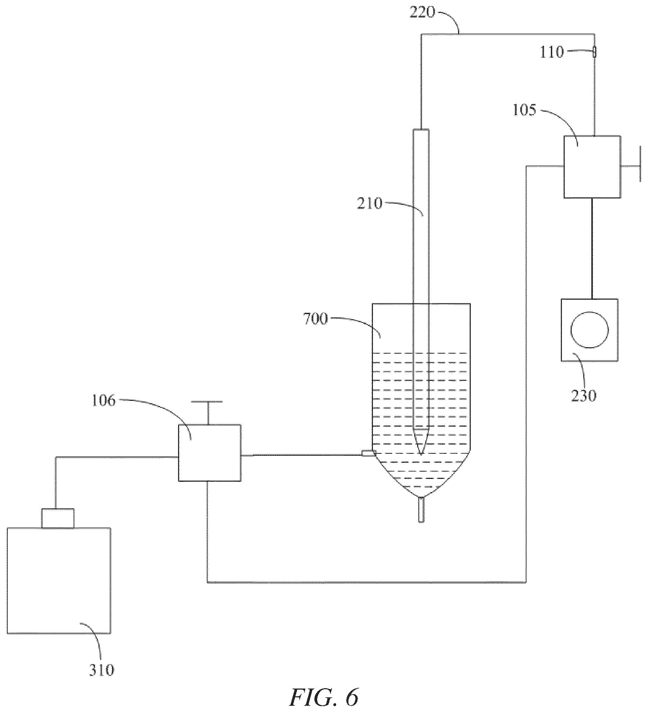

Referring to FIG. 1 and FIGS. 4 to 6, the blood sample analyzer 10 and the control method therefor according to this embodiment are different from the second embodiment and the third embodiment mainly in that, in the second embodiment and the third embodiment, the delivery of the blood sample and the delivery of the alkaline cleaning liquid are driven by the same power source, and no cleaning cell 700 is provided; while in this embodiment, although the delivery of the blood sample and the delivery of the alkaline cleaning liquid are driven by the same power source, a cleaning cell 700 is provided in this embodiment.

Specifically, in this embodiment, the blood sample analyzer 10 further includes a cleaning cell 700 and a valve group, and the first cleaning liquid supply device 300 includes a first liquid storage container 310, the first liquid storage container 310 being configured to store the alkaline cleaning liquid; and the first power source 230 is switchable to be connected to the sample aspiration pipe 220, the first liquid storage container 310 and the cleaning cell 700 by means of the valve group.

As an implementation of this embodiment, the analysis and control device 400 is further configured to:
control the valve group to switch to a state in which the first power source 230 is in conduction with the first liquid storage container 310, and control the first power source 230 to drive aspiration of the alkaline cleaning liquid from the first liquid storage container 310;
control the valve group to switch to a state in which the first power source 230 is in conduction with the cleaning cell 700, and control the first power source 230 to deliver the aspirated alkaline cleaning liquid to the cleaning cell 700; and
control the valve group to switch to a state in which the first power source 230 is in conduction with the sample aspiration pipe 220, and control the first power source 230 to drive the sample collecting needle 210 to aspirate the alkaline cleaning liquid from the cleaning cell 700 and deliver the alkaline cleaning liquid to the ESR test pipe 110, so as to clean the ESR test pipe 110.

As an implementation, the valve group includes a seventh control valve 105 and an eighth control valve 106, each of the seventh control valve 105 and the eighth control valve 106 being a three-way valve. The first power source 230 is switchable to be connected to the ESR test pipe 110 and the eighth control valve 106 by means of the seventh control valve 105, that is, three ports of the seventh control valve 105 are respectively connected to the first power source 230, the ESR test pipe 110 and the eighth control valve 106. The seventh control valve 105 is switchable to be connected to the first liquid storage container 310 and the cleaning cell 700 by means of the eighth control valve 106, that is, three ports of the eighth control valve 106 are respectively connected to the first liquid storage container 310, the cleaning cell 700 and the seventh control valve 105. Certainly, in the specific application, the valve group is arranged in a manner not limited thereto. For example, as an alternative embodiment, the seventh control valve 105 and the eighth control valve 106 can be replaced with two two-way valves.

The differences from the first embodiment lies in that, in the method for controlling the blood sample analyzer 10 according to this embodiment, controlling delivery of the alkaline cleaning liquid supplied by the first cleaning liquid supply device 300 to the ESR test pipe 110 includes:
controlling the valve group to switch to a state in which the first power source 230 is in conduction with the first liquid storage container 310, and controlling the first power source 230 to drive aspiration of the alkaline cleaning liquid from the first liquid storage container 310;
controlling the valve group to switch to a state in which the first power source 230 is in conduction with the cleaning cell 700, and controlling the first power source 230 to deliver the aspirated alkaline cleaning liquid to the cleaning cell 700; and
controlling the valve group to switch to a state in which the first power source 230 in conduction with the sample aspiration pipe 220, and controlling the first power source 230 to drive the sample collecting needle 210 to aspirate the alkaline cleaning liquid from the cleaning cell 700 and deliver the alkaline cleaning liquid to the ESR test pipe 110.

In addition to the differences described above, other content of the blood sample analyzer 10 and the control method therefor according to this embodiment may be optimally designed with reference to the first embodiment to the third embodiment, which will not be described in detail herein.

### Fifth Embodiment:

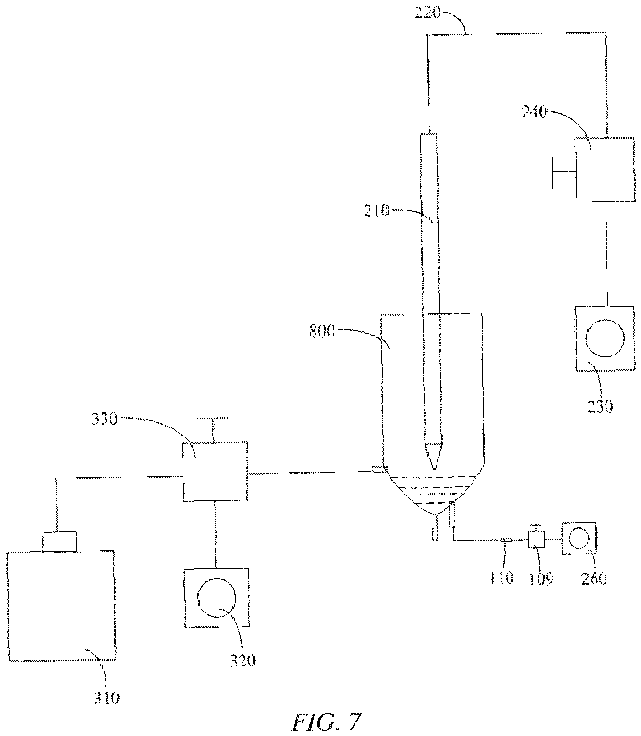

Referring to FIGS. 1, 2 and 7, the blood sample analyzer 10 and the control method therefor according to this embodiment are different from those in the first embodiment to the forth embodiment mainly in the different methods for the arrangement of the ESR test pipe 110, which specifically lies in that, in the first embodiment to the forth embodiment, the ESR test pipe 110 is connected to or integrated with the sample aspiration pipe 220; while in this embodiment, the ESR test pipe 110 is neither connected to the sample aspiration pipe 220, nor integrated with the sample aspiration pipe 220.

As an implementation, in this embodiment, the blood sample analyzer 10 further includes a temporary storage cell 800, and the sample collecting and dispensing device 200 includes a sample collecting needle 210, a sample aspiration pipe 220, a first power source 230 and a third power source 260, the sample aspiration pipe 220 being connected to the sample collecting needle 210 and the first power source 230 respectively, and the ESR test pipe 110 being connected to the temporary storage cell 800 and the third power source 260 respectively.

As an implementation, in this embodiment, the analysis and control device 400 is further configured to control the first power source 230 to drive the sample collecting needle 210 to aspirate the blood sample from the sample container 20, control the first power source 230 to drive discharge of at least part of the blood sample aspirated by the sample collecting needle 210 to the temporary storage cell 800, and control the third power source 260 to drive delivery of at least part of the blood sample in the temporary storage cell 800 to the ESR test pipe 110.

As an implementation, a ninth control valve 109 is further provided between the temporary storage cell 800 and the third power source 260 for controlling connection and disconnection of the ESR test pipe 110.

As an implementation, the ESR test pipe 110 may be of one-piece, that is, pipe connected between the temporary storage cell 800 and the third power source 260 are all capillary tubes. Certainly, in an alternative embodiment, the ESR test pipe 110 may also be divided into at least two segments, one of which is a capillary tube for the ESR test.

As an implementation, the first cleaning liquid supply device 300 includes a first liquid storage container 310 and a second power source 320, the first liquid storage container 310 being configured to store the alkaline cleaning liquid, and the second power source 320 being connected to the first liquid storage container 310 and the temporary storage cell 800 respectively. The analysis and control device 400 is further configured to control, after completion of the ESR test in the ESR test pipe 110, the second power source 320 to drive delivery of the alkaline cleaning liquid in the first liquid storage container 310 to the temporary storage cell 800, and control the third power source 260 to drive delivery of the alkaline cleaning liquid in the temporary storage cell 800 to the ESR test pipe 110, so as to clean the temporary storage cell 800 and the ESR test pipe 110.

Different from the first embodiment, in the method for controlling the blood sample analyzer 10 according to this embodiment, controlling the sample collecting and dispensing device 200 to aspirate the blood sample from the sample container 20 includes: controlling the first power source 230 to drive the sample collecting needle 210 to aspirate the blood sample from the sample container 20. Controlling the sample collecting and dispensing device 200 to dispense at least part of the aspirated blood sample to the ESR test pipe 110 includes: controlling the first power source 230 to drive discharge of at least part of the blood sample aspirated by the sample collecting needle 210 to the temporary storage cell 800, and controlling the third power source 260 to drive delivery of at least part of the blood sample in the temporary storage cell 800 to the ESR test pipe 110.

In the method for controlling the blood sample analyzer 10 according to this embodiment, controlling delivery of the alkaline cleaning liquid supplied by the first cleaning liquid supply device 300 to the ESR test pipe 110 includes: controlling the second power source 320 to drive delivery of the alkaline cleaning liquid in the first liquid storage container 310 to the temporary storage cell 800, and controlling the third power source 260 to drive delivery of the alkaline cleaning liquid in the temporary storage cell 800 to the ESR test pipe 110.

In addition to the differences described above, other content of the blood sample analyzer 10 and the control method therefor according to this embodiment may be optimally designed with reference to the first embodiment to the fourth embodiment, which will not be described in detail herein.

### Six Embodiment:

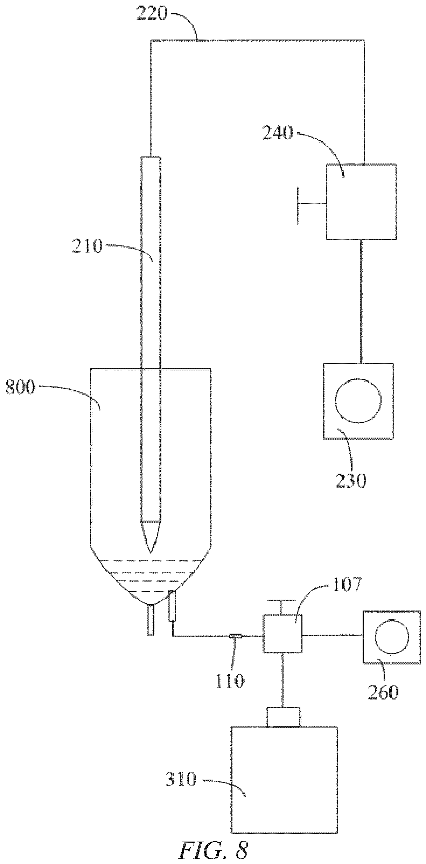

Referring to FIGS. 1, 7 and 8, the blood sample analyzer 10 and the control method therefor according to this embodiment are different from those in the fifth embodiment mainly in the manner of driving the delivery of the alkaline cleaning liquid, which specifically lies in that, in the fifth embodiment, the delivery of the blood sample and the delivery of the alkaline cleaning liquid are driven by three mutually independent power sources, one of which is configured to collect a sample and dispense the sample to the temporary storage cell 800, one is configured to deliver the alkaline cleaning liquid to the temporary storage cell 800, and the other is configured to aspirate the blood sample from the temporary storage cell 800 to the ESR test pipe 110 and aspirate the alkaline cleaning liquid from the temporary storage cell 800 to the ESR test pipe 110; while in this embodiment, the delivery of the blood sample and the delivery of the alkaline cleaning liquid are driven by two power sources, one of which is configured to drive delivery of the blood sample and the alkaline cleaning liquid between the temporary storage cell 800 and the ESR test pipe 110, and the other is configured to drive the sample collecting needle 210 to collect a sample and dispense the sample.

Specifically, in this embodiment, the first cleaning liquid supply device 300 also includes a first liquid storage container 310. The first liquid storage container 310 is configured to store the alkaline cleaning liquid. The blood sample analyzer 10 further includes a second control valve 107, and the third power source 260 is switchable to be connected to the ESR test pipe 110 and the first liquid storage container 310 by means of the second control valve 107.

The analysis and control device 400 is further configured to control, after completion of the ESR test in the ESR test pipe 110, the second control valve 107 to switch to a state in which the third power source 260 is in conduction with the first liquid storage container 310, then control the third power source 260 to drive aspiration of the alkaline cleaning liquid from the first liquid storage container 310, then control the second control valve 107 to switch to a state in which the third power source 260 is in conduction with the ESR test pipe 110, and control the third power source 260 to drive delivery of the aspirated alkaline cleaning liquid to the ESR test pipe 110 and the temporary storage cell 800, so as to clean the ESR test pipe 110 and the temporary storage cell 800.

In the method for controlling the blood sample analyzer 10 according to this embodiment, controlling delivery of the alkaline cleaning liquid supplied by the first cleaning liquid supply device 300 to the ESR test pipe 110 includes:
controlling the second control valve 107 to switch to a state in which the third power source 260 is in conduction with the first liquid storage container 310, and controlling the third power source 260 to drive aspiration of the alkaline cleaning liquid from the first liquid storage container 310; and
controlling the second control valve 107 to switch to a state in which the third power source 260 is in conduction with the ESR test pipe 110, and controlling the third power source 260 to drive delivery of the alkaline cleaning liquid from the first liquid storage container 310 to the ESR test pipe 110 and the temporary storage cell 800.

In addition to the differences described above, other content of the blood sample analyzer 10 and the control method therefor according to this embodiment may be optimally designed with reference to the first embodiment to the fifth embodiment, which will not be described in detail herein.

### Seventh Embodiment:

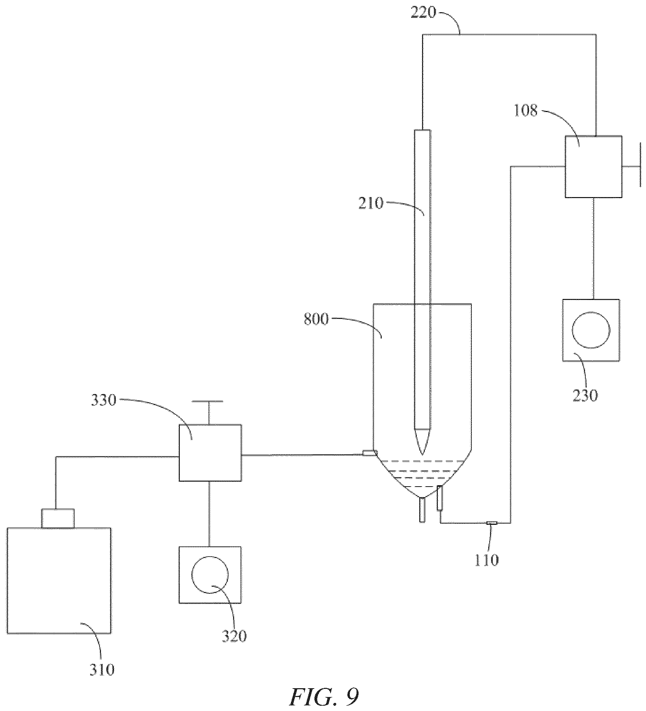

Referring to FIGS. 1, 8 and 9, the blood sample analyzer 10 and the control method therefor according to this embodiment are different from those in the sixth embodiment mainly in the manner of driving the delivery of the alkaline cleaning liquid, which specifically lies in that, in the sixth embodiment, the delivery of the blood sample and the delivery of the alkaline cleaning liquid are driven by two power sources, one of which is configured to drive delivery of the blood sample and the alkaline cleaning liquid between the temporary storage cell 800 and the ESR test pipe 110, and the other is configured to drive the sample collecting needle 210 to collect a sample and dispense the sample; while in this embodiment, one power source is provided, and is configured to drive delivery of the blood sample and the alkaline cleaning liquid between the temporary storage cell 800 and the ESR test pipe 110 and drive the sample collecting needle 210 to collect a sample and dispense the sample.

In this embodiment, the blood sample analyzer 10 further includes a temporary storage cell 800 and a third control valve 108; the sample collecting and dispensing device 200 includes a sample collecting needle 210, a sample aspiration pipe 220 and a first power source 230, the sample aspiration pipe 220 being connected to the sample collecting needle 210 and the first power source 230 respectively, and the ESR test pipe 110 being connected to the temporary storage cell 800 and the first power source 230 respectively; and the first power source 230 is switchable to be connected to the sample aspiration pipe 220 and the ESR test pipe 110 by means of the third control valve 108. The third control valve 108 may be a three-way valve or may include two two-way valves.

The analysis and control device 400 is further configured to control the third control valve 108 to switch to a state in which the first power source 230 is in conduction with the sample aspiration pipe 220, control the first power source 230 to drive the sample collecting needle 210 to aspirate the blood sample from the sample container 20, control the first power source 230 to discharge at least part of the blood sample aspirated by the sample collecting needle 210 to the temporary storage cell 800, control the third control valve 108 to switch to a state in which the first power source 230 is in conduction with the ESR test pipe 110, and control the first power source 230 to drive delivery of at least part of the blood sample in the temporary storage cell 800 to the ESR test pipe 110.

In the method for controlling the blood sample analyzer 10 according to this embodiment, controlling the sample collecting and dispensing device 200 to aspirate the blood sample from the sample container 20 includes: controlling the third control valve 108 to switch to a state in which the first power source 230 is in conduction with the sample aspiration pipe 220, and controlling the first power source 230 to drive the sample collecting needle 210 to aspirate the blood sample from the sample container 20.

In the method for controlling the blood sample analyzer 10 according to this embodiment, controlling the sample collecting and dispensing device 200 to dispense at least part of the aspirated blood sample to the ESR test pipe 110 includes: controlling the first power source 230 to drive discharge of the at least part of the blood sample aspirated by the sample collecting needle 210 to the temporary storage cell 800, controlling the third control valve 108 to switch to a state in which the first power source 230 is in conduction with the ESR test pipe 110, and controlling the first power source 230 to drive delivery of at least part of the blood sample in the temporary storage cell 800 to the ESR test pipe 110.

In addition to the differences described above, other content of the blood sample analyzer 10 and the control method therefor according to this embodiment may be optimally designed with reference to the first embodiment to the sixth embodiment, which will not be described in detail herein.

### Eighth Embodiment:

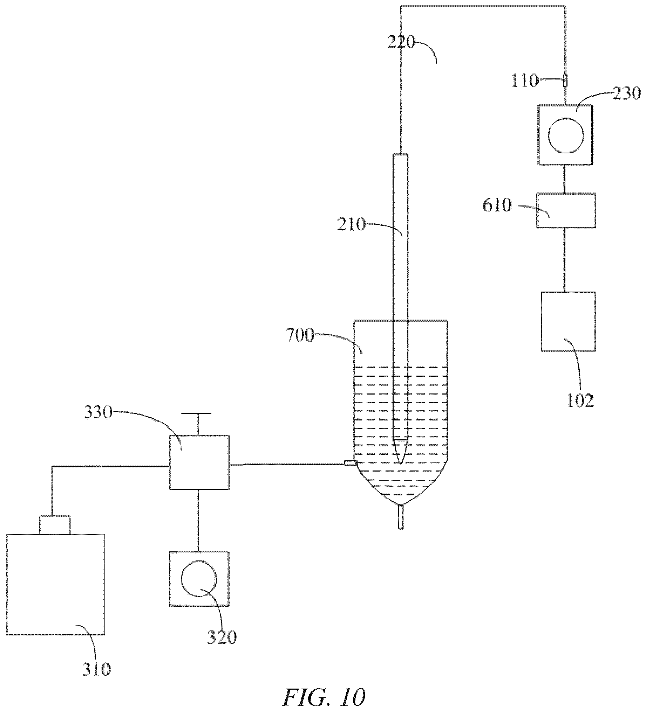

Referring to FIGS. 1 to 3 and FIG.10, the blood sample analyzer 10 and the control method therefor according to this embodiment are different from those in the first embodiment mainly in the manner of sample dispensing for the blood routine test, which specifically lies in that, in the first embodiment, there is no connection relationship between the test cell 610 or the reaction cell and the sample aspiration pipe 220, and the sample collecting and dispensing device 200 dispenses the collected blood sample to the test cell 610 or the reaction cell by means of the sample collecting needle 210; while in this embodiment, the test cell 610 or the reaction cell is connected to the sample aspiration pipe 220 and the ESR test pipe 110, and the sample collecting and dispensing device 200 dispenses the blood sample after completion of the ESR test to the test cell 610 or the reaction cell by means of the sample aspiration pipe 220 and the ESR test pipe 110, so as to continue to perform the blood routine test.

Specifically, in this embodiment, the sample aspiration pipe 220 is connected to the sample collecting needle 210, the first power source 230 and the test cell 610 (or the reaction cell) respectively, and the ESR test pipe 110 is located between the sample collecting needle 210 and the test cell 610 (or between the sample collecting needle 210 and the reaction cell). The analysis and control device 400 is configured to control the first power source 230 to deliver at least part of the blood sample aspirated by the sample collecting needle 210 to the ESR test pipe 110 to perform the ESR test and then control, after completion of the ESR test, the first power source 230 to deliver the blood sample in the ESR test pipe 110 to the test cell 610 to perform a blood routine test, or deliver the blood sample to the reaction cell to prepare a test sample and then deliver the test sample to the test cell 610 to perform a blood routine test. According to this embodiment, the blood consumption can be reduced.

In addition to the differences described above, other content of the blood sample analyzer 10 and the control method therefor according to this embodiment may be optimally designed with reference to the first embodiment to the seventh embodiment, which will not be described in detail herein.

The foregoing description merely relates to the preferred embodiments of the disclosure, and is not intended to limit the scope of patent of the disclosure. All equivalent structural variations made according to the content in the specification and the accompanying drawings of the disclosure from the concept of the disclosure, or the direct/indirect applications of the contents in other related technical fields shall all fall within the scope of patent protection of the disclosure.

### Examples

1. A blood sample analyzer, characterized in that the blood sample analyzer comprises:
   an ESR test device comprising an ESR test pipe and a photoelectric measurement component, the ESR test pipe being configured to provide a test place for erythrocyte aggregation of a blood sample, and the photoelectric measurement component being configured to measure a level of erythrocyte aggregation of the blood sample in the ESR test pipe;
   a sample collecting and dispensing device configured to aspirate the blood sample from a sample container and dispense at least part of the aspirated blood sample to the ESR test pipe;
   a first cleaning liquid supply device configured to supply an alkaline cleaning liquid; and
   an analysis and control device configured to:
      analyze measurement data related to the level of erythrocyte aggregation measured by the photoelectric measurement component to obtain an erythrocyte sedimentation rate of the blood sample; and
      control delivery of the alkaline cleaning liquid supplied by the first cleaning liquid supply device to the ESR test pipe after completion of an ESR test in the ESR test pipe, so as to clean the ESR test pipe.
2. The blood sample analyzer of example 1, characterized in that the sample collecting and dispensing device comprises a sample collecting needle, a sample aspiration pipe and a first power source, the sample aspiration pipe being connected to the sample collecting needle and the first power source respectively;
   the ESR test pipe is connected to the sample aspiration pipe, or a portion of the sample aspiration pipe is used as the ESR test pipe; and
   the analysis and control device is further configured to control the first power source to drive the sample collecting needle to aspirate the blood sample from the sample container, and control the first power source to drive the sample collecting needle to deliver the at least part of the aspirated blood sample to the ESR test pipe.
3. The blood sample analyzer of example 2, characterized in that the blood sample analyzer further comprises a cleaning cell, and the first cleaning liquid supply device comprises a first liquid storage container and a second power source, the first liquid storage container being configured to store the alkaline cleaning liquid, and the second power source being connected to the first liquid storage container and the cleaning cell respectively; and
   the analysis and control device is further configured to control the second power source to drive delivery of the alkaline cleaning liquid in the first liquid storage container to the cleaning cell, and control, after completion of the ESR test in the ESR test pipe, the first power source to drive the sample collecting needle to aspirate the alkaline cleaning liquid from the cleaning cell and deliver the alkaline cleaning liquid to the ESR test pipe to clean the ESR test pipe.
4. The blood sample analyzer of example 2, characterized in that the first cleaning liquid supply device comprises a first liquid storage container configured to store the alkaline cleaning liquid;
   the blood sample analyzer further comprises a first control valve, through which the first power source is switchable to be connected to the sample aspiration pipe and the first liquid storage container; and
   the analysis and control device is further configured to, after completion of the ESR test in the ESR test pipe, control the first control valve to switch to a state in which the first power source is in conduction with the first liquid storage container, then control the first power source to aspirate the alkaline cleaning liquid from the first liquid storage container, then control the first control valve to switch to a state in which the first power source is in conduction with the sample aspiration pipe, and control the first power source to deliver the aspirated alkaline cleaning liquid to the ESR test pipe, so as to clean the ESR test pipe.
5. The blood sample analyzer of example 4, characterized in that the blood sample analyzer further comprises a waste liquid cell and a first liquid discharge pipe that is connected between the waste liquid cell and the first power source, and the analysis and control device is further configured to control the first power source to drive delivery of the cleaning liquid after cleaning of the ESR test pipe to the waste liquid cell via the first liquid discharge pipe; or
   the blood sample analyzer further comprises a waste liquid cell, and the analysis and control device is further configured to control the first power source to drive discharge of the cleaning liquid after cleaning of the ESR test pipe to the waste liquid cell via the sample aspiration pipe and the sample collecting needle.
6. The blood sample analyzer of example 2, characterized in that the blood sample analyzer further comprises a cleaning cell and a valve group, and the first cleaning liquid supply device comprises a first liquid storage container configured to store the alkaline cleaning liquid;
   the first power source is switchable to be connected to the sample aspiration pipe, the first liquid storage container and the cleaning cell by means of the valve group; and
   the analysis and control device is further configured to:
      control the valve group to switch to a state in which the first power source is in conduction with the first liquid storage container, and control the first power source to aspirate the alkaline cleaning liquid from the first liquid storage container;
      control the valve group to switch to a state in which the first power source is in conduction with the cleaning cell, and control the first power source to deliver the aspirated alkaline cleaning liquid to the cleaning cell; and
      control the valve group to switch to a state in which the first power source is in conduction with the sample aspiration pipe, and control the first power source to drive the sample collecting needle to aspirate the alkaline cleaning liquid from the cleaning cell and deliver the alkaline cleaning liquid to the ESR test pipe, so as to clean the ESR test pipe.
7. The blood sample analyzer of example 1, characterized in that the blood sample analyzer further comprises a temporary storage cell;
   the sample collecting and dispensing device comprises a sample collecting needle, a sample aspiration pipe, a first power source and a third power source, the sample aspiration pipe being connected to the sample collecting needle and the first power source respectively, and the ESR test pipe being connected to the temporary storage cell and the third power source respectively; and
   the analysis and control device is further configured to control the first power source to drive the sample collecting needle to aspirate the blood sample from the sample container, control the first power source to drive discharge of at least part of the blood sample aspirated by the sample collecting needle to the temporary storage cell, and control the third power source to drive delivery of at least part of the blood sample in the temporary storage cell to the ESR test pipe.
8. The blood sample analyzer of example 7, characterized in that the first cleaning liquid supply device comprises a first liquid storage container and a second power source, the first liquid storage container being configured to store the alkaline cleaning liquid, and the second power source being connected to the first liquid storage container and the temporary storage cell respectively; and
   the analysis and control device is further configured to control, after completion of the ESR test in the ESR test pipe, the second power source to drive delivery of the alkaline cleaning liquid in the first liquid storage container to the temporary storage cell, and control the third power source to drive delivery of the alkaline cleaning liquid in the temporary storage cell to the ESR test pipe, so as to clean the temporary storage cell and the ESR test pipe.
9. The blood sample analyzer of example 7, characterized in that the first cleaning liquid supply device comprises a first liquid storage container configured to store the alkaline cleaning liquid;
   the blood sample analyzer further comprises a second control valve, through which the third power source is switchable to be connected to the ESR test pipe and the first liquid storage container; and
   the analysis and control device is further configured to, after completion of the ESR test in the ESR test pipe, control the second control valve to switch to a state in which the third power source is in conduction with the first liquid storage container, then control the third power source to drive aspiration of the alkaline cleaning liquid from the first liquid storage container, then control the second control valve to switch to a state in which the third power source is in conduction with the ESR test pipe, and control the third power source to drive delivery of the alkaline cleaning liquid to the ESR test pipe and the temporary storage cell, so as to clean the ESR test pipe and the temporary storage cell.
10. The blood sample analyzer of example 1, characterized in that the blood sample analyzer further comprises a temporary storage cell and a third control valve;
   the sample collecting and dispensing device comprises a sample collecting needle, a sample aspiration pipe and a first power source, the sample aspiration pipe being connected to the sample collecting needle and the first power source respectively, and the ESR test pipe being connected to the temporary storage cell and the first power source respectively;
   the first power source is switchable to be connected to the sample aspiration pipe and the ESR test pipe by means of the third control valve; and
   the analysis and control device is further configured to control the third control valve to switch to a state in which the first power source is in conduction with the sample aspiration pipe, control the first power source to drive the sample collecting needle to aspirate the blood sample from the sample container, control the first power source to drive discharge of at least part of the blood sample aspirated by the sample collecting needle to the temporary storage cell, control the third control valve to switch to a state in which the first power source is in conduction with the ESR test pipe, and control the first power source to drive delivery of at least part of the blood sample in the temporary storage cell to the ESR test pipe.
11. The blood sample analyzer of example 1, characterized in that the blood sample analyzer further comprises a blood routine test device and a second cleaning liquid supply device that is configured to supply a neutral cleaning liquid;
   the blood routine test device comprises a test cell and a blood routine test component, the sample collecting and dispensing device is further configured to dispense at least part of the aspirated blood sample to the test cell or to a reaction cell that is connected to the test cell by means of a pipe, and the blood routine test component is configured to perform a blood routine test on the blood sample in the test cell, the blood routine test comprising at least one of differential leukocyte count, reticulocyte count, hemoglobin measurement, erythrocyte count and platelet count; and
   the analysis and control device is further configured to control delivery of the neutral cleaning liquid supplied by the second cleaning liquid supply device to the test cell after completion of the blood routine test in the test cell, so as to clean the test cell.
12. The blood sample analyzer of example 11, characterized in that the sample collecting and dispensing device comprises a sample collecting needle, a sample aspiration pipe and a first power source;
   the ESR test pipe is connected to the sample aspiration pipe, or a portion of the sample aspiration pipe is used as the ESR test pipe;
   the sample aspiration pipe is connected to the sample collecting needle, the first power source and the test cell respectively, the ESR test pipe being located between the sample collecting needle and the test cell, or the sample aspiration pipe is connected to the sample collecting needle, the first power source and the reaction cell respectively, the ESR test pipe being located between the sample collecting needle and the reaction cell; and
   the analysis and control device is configured to control the first power source to deliver at least part of the blood sample aspirated by the sample collecting needle to the ESR test pipe to perform the ESR test, and then control, after completion of the ESR test, the first power source to deliver the blood sample in the ESR test pipe to the test cell or the reaction cell.
13. The blood sample analyzer of example 11, characterized in that the sample collecting and dispensing device comprises a sample collecting needle, a sample aspiration pipe and a first power source;
   the ESR test pipe is connected to the sample aspiration pipe, or a portion of the sample aspiration pipe is used as the ESR test pipe;
   the sample aspiration pipe is connected to the sample collecting needle and the first power source respectively; and
   the analysis and control device is configured to control the first power source to dispense at least part of the blood sample aspirated by the sample collecting needle to the test cell or the reaction cell via the sample collecting needle, and then control the first power source to deliver at least part of the blood sample aspirated by the sample collecting needle to the ESR test pipe; or control the first power source to deliver at least part of the blood sample aspirated by the sample collecting needle to the ESR test pipe, and then control the first power source to dispense at least part of the blood sample aspirated by the sample collecting needle to the test cell or the reaction cell via the sample collecting needle.
14. The blood sample analyzer of any one of examples 1 to 13, characterized in that the blood sample analyzer further comprises a second cleaning liquid supply device configured to supply a neutral cleaning liquid; and
   the analysis and control device is further configured to: after completion of the ESR test in the ESR test pipe, deliver the alkaline cleaning liquid supplied by the first cleaning liquid supply device to the ESR test pipe to clean the ESR test pipe, and then deliver the neutral cleaning liquid supplied by the second cleaning liquid supply device to the ESR test pipe to clean the ESR test pipe.
15. The blood sample analyzer of example 14, characterized in that, after completion of one ESR test, the ESR test pipe is sequentially cleaned with the alkaline cleaning liquid and the neutral cleaning liquid, so as to ensure that the ESR test pipe does not affect measurement of a level of erythrocyte aggregation of a next blood sample.
16. The blood sample analyzer of any one of examples 1 to 13, characterized in that the alkaline cleaning liquid contains at least one surfactant.
17. The blood sample analyzer of any one of examples 1 to 13, characterized in that the ESR test pipe is a capillary tube.
18. A method for controlling a blood sample analyzer, characterized in that the method comprises:
   controlling a sample collecting and dispensing device to aspirate a blood sample from a sample container;
   controlling the sample collecting and dispensing device to dispense at least part of the aspirated blood sample to an ESR test pipe;
   controlling a photoelectric measurement component to measure a level of erythrocyte aggregation of the blood sample in the ESR test pipe;
   analyzing measurement data related to the level of erythrocyte aggregation measured by the photoelectric measurement component to obtain an erythrocyte sedimentation rate of the blood sample; and
   controlling delivery of an alkaline cleaning liquid supplied by a first cleaning liquid supply device to the ESR test pipe, so as to clean the ESR test pipe.
19. The method for controlling a blood sample analyzer of example 18, characterized in that the control method further comprises, after the ESR test pipe is cleaned with the alkaline cleaning liquid, controlling delivery of a neutral cleaning liquid supplied by a second cleaning liquid supply device to the ESR test pipe, so as to clean the ESR test pipe.
20. The method for controlling a blood sample analyzer of example 18 or 19, characterized in that the control method further comprises, before or after the sample collecting and dispensing device is controlled to dispense the aspirated blood sample to the ESR test pipe,
   controlling the sample collecting and dispensing device to dispense at least part of the aspirated blood sample to a test cell or to a reaction cell that is connected to the test cell by means of a pipe;
   controlling a blood routine test component to perform a blood routine test on the blood sample in the test cell, the blood routine test comprising at least one of differential leukocyte count, reticulocyte count, hemoglobin measurement, erythrocyte count and platelet count;
   analyzing and processing test data of the blood routine test component to obtain blood routine test parameters of the blood sample; and
   controlling delivery of a/the neutral cleaning liquid supplied by a/the second cleaning liquid supply device to the test cell, so as to clean the test cell.
21. The method for controlling a blood sample analyzer of example 18 or 19, characterized in that
   controlling a sample collecting and dispensing device to aspirate a blood sample from a sample container comprises: controlling a first power source to drive a sample collecting needle to aspirate the blood sample from the sample container; and
   controlling the sample collecting and dispensing device to dispense at least part of the aspirated blood sample to an ESR test pipe comprises: controlling the first power source to drive the sample collecting needle to deliver the at least part of the aspirated blood sample to the ESR test pipe,
   wherein the sample collecting needle is connected to the first power source by means of a sample aspiration pipe; and
   the ESR test pipe is connected to the sample aspiration pipe, or a portion of the sample aspiration pipe is used as the ESR test pipe.
22. The method for controlling a blood sample analyzer of example 21, characterized in that controlling delivery of an alkaline cleaning liquid supplied by a first cleaning liquid supply device to the ESR test pipe comprises: controlling a second power source to drive delivery of the alkaline cleaning liquid in a first liquid storage container to a cleaning cell; and controlling the first power source to drive the sample collecting needle to aspirate the alkaline cleaning liquid from the cleaning cell and deliver the alkaline cleaning liquid to the ESR test pipe.
23. The method for controlling a blood sample analyzer of example 21, characterized in that controlling delivery of an alkaline cleaning liquid supplied by a first cleaning liquid supply device to the ESR test pipe comprises:
   controlling a first control valve to switch to a state in which the first power source is in conduction with a first liquid storage container, and controlling the first power source to aspirate the alkaline cleaning liquid from the first liquid storage container; and
   controlling the first control valve to switch to a state in which the first power source is in conduction with the sample aspiration pipe, and controlling the first power source to deliver the aspirated alkaline cleaning liquid to the ESR test pipe.
24. The method for controlling a blood sample analyzer of example 18 or 19, characterized in that controlling delivery of an alkaline cleaning liquid supplied by a first cleaning liquid supply device to the ESR test pipe comprises:
   controlling a valve group to switch to a state in which a first power source is in conduction with a first liquid storage container, and controlling the first power source to aspirate the alkaline cleaning liquid from the first liquid storage container;
   controlling the valve group to switch to a state in which the first power source is in conduction with a cleaning cell, and controlling the first power source to deliver the aspirated alkaline cleaning liquid to the cleaning cell; and
   controlling the valve group to switch to a state in which the first power source is in conduction with a sample aspiration pipe, and controlling the first power source to drive a sample collecting needle to aspirate the alkaline cleaning liquid from the cleaning cell and deliver the alkaline cleaning liquid to the ESR test pipe,
   wherein the sample aspiration pipe is connected to the sample collecting needle and the first power source respectively; and
   the ESR test pipe is connected to the sample aspiration pipe, or a portion of the sample aspiration pipe is used as the ESR test pipe.
25. The method for controlling a blood sample analyzer of example 18 or 19, characterized in that controlling a sample collecting and dispensing device to aspirate a blood sample from a sample container comprises: controlling a first power source to drive a sample collecting needle to aspirate the blood sample from the sample container; and
   controlling the sample collecting and dispensing device to dispense at least part of the aspirated blood sample to an ESR test pipe comprises: controlling the first power source to drive discharge of the at least part of the blood sample aspirated by the sample collecting needle to a temporary storage cell, and controlling a third power source to drive delivery of at least part of the blood sample in the temporary storage cell to the ESR test pipe,
   wherein the sample collecting needle is connected to the first power source by means of a sample aspiration pipe; and
   the ESR test pipe is connected to the temporary storage cell and the third power source respectively.
26. The method for controlling a blood sample analyzer of example 25, characterized in that controlling an alkaline cleaning liquid supplied by a first cleaning liquid supply device to the ESR test pipe comprises: controlling a second power source to drive delivery of the alkaline cleaning liquid in a first liquid storage container to the temporary storage cell, and controlling the third power source to drive delivery of the alkaline cleaning liquid in the temporary storage cell to the ESR test pipe.
27. The method for controlling a blood sample analyzer of example 25, characterized in that controlling an alkaline cleaning liquid supplied by a first cleaning liquid supply device to the ESR test pipe comprises:
   controlling a second control valve to switch to a state in which the third power source is in conduction with a first liquid storage container, and controlling the third power source to drive aspiration of the alkaline cleaning liquid from the first liquid storage container; and
   controlling the second control valve to switch to a state in which the third power source is in conduction with the ESR test pipe, and controlling the third power source to drive delivery of the alkaline cleaning liquid from the first liquid storage container to the ESR test pipe and the temporary storage cell.
28. The method for controlling a blood sample analyzer of example 18 or 19, characterized in that
   controlling a sample collecting and dispensing device to aspirate a blood sample from a sample container comprises: controlling a third control valve to switch to a state in which a first power source is in conduction with a sample aspiration pipe, and controlling the first power source to drive a sample collecting needle to aspirate the blood sample from the sample container; and
   controlling the sample collecting and dispensing device to dispense at least part of the aspirated blood sample to an ESR test pipe comprises: controlling the first power source to drive discharge of the at least part of the blood sample aspirated by the sample collecting needle to a temporary storage cell, controlling the third control valve to switch to a state in which the first power source is in conduction with the ESR test pipe, and controlling the first power source to drive delivery of at least part of the blood sample in the temporary storage cell to the ESR test pipe,
   wherein the sample aspiration pipe is connected to the sample collecting needle, and is connected to the first power source by means of the third control valve; and
   the ESR test pipe is connected to the temporary storage cell, and is connected to the first power source by means of the third control valve.

## Claims

1. A blood sample analyzer, **characterized in that** the blood sample analyzer comprises:
an ESR test device comprising an ESR test pipe and a photoelectric measurement component, the ESR test pipe being configured to provide a test place for erythrocyte aggregation of a blood sample, and the photoelectric measurement component being configured to measure a level of erythrocyte aggregation of the blood sample in the ESR test pipe;
a sample collecting and dispensing device configured to aspirate the blood sample from a sample container and dispense at least part of the aspirated blood sample to the ESR test pipe;
a first cleaning liquid supply device configured to supply an alkaline cleaning liquid; and
an analysis and control device configured to:
analyze measurement data related to the level of erythrocyte aggregation measured by the photoelectric measurement component to obtain an erythrocyte sedimentation rate of the blood sample; and
control delivery of the alkaline cleaning liquid supplied by the first cleaning liquid supply device to the ESR test pipe after completion of an ESR test in the ESR test pipe, so as to clean the ESR test pipe.

2. The blood sample analyzer of claim 1, **characterized in that** the sample collecting and dispensing device comprises a sample collecting needle, a sample aspiration pipe and a first power source, the sample aspiration pipe being connected to the sample collecting needle and the first power source respectively;
the ESR test pipe is connected to the sample aspiration pipe, or a portion of the sample aspiration pipe is used as the ESR test pipe; and
the analysis and control device is further configured to control the first power source to drive the sample collecting needle to aspirate the blood sample from the sample container, and control the first power source to drive the sample collecting needle to deliver the at least part of the aspirated blood sample to the ESR test pipe.

3. The blood sample analyzer of claim 2, **characterized in that** the blood sample analyzer further comprises a cleaning cell, and the first cleaning liquid supply device comprises a first liquid storage container and a second power source, the first liquid storage container being configured to store the alkaline cleaning liquid, and the second power source being connected to the first liquid storage container and the cleaning cell respectively; and
the analysis and control device is further configured to control the second power source to drive delivery of the alkaline cleaning liquid in the first liquid storage container to the cleaning cell, and control, after completion of the ESR test in the ESR test pipe, the first power source to drive the sample collecting needle to aspirate the alkaline cleaning liquid from the cleaning cell and deliver the alkaline cleaning liquid to the ESR test pipe to clean the ESR test pipe.

4. The blood sample analyzer of claim 2, **characterized in that** the first cleaning liquid supply device comprises a first liquid storage container configured to store the alkaline cleaning liquid;
the blood sample analyzer further comprises a first control valve, through which the first power source is switchable to be connected to the sample aspiration pipe and the first liquid storage container; and
the analysis and control device is further configured to, after completion of the ESR test in the ESR test pipe, control the first control valve to switch to a state in which the first power source is in conduction with the first liquid storage container, then control the first power source to aspirate the alkaline cleaning liquid from the first liquid storage container, then control the first control valve to switch to a state in which the first power source is in conduction with the sample aspiration pipe, and control the first power source to deliver the aspirated alkaline cleaning liquid to the ESR test pipe, so as to clean the ESR test pipe.

5. The blood sample analyzer of claim 4, **characterized in that** the blood sample analyzer further comprises a waste liquid cell and a first liquid discharge pipe that is connected between the waste liquid cell and the first power source, and the analysis and control device is further configured to control the first power source to drive delivery of the cleaning liquid after cleaning of the ESR test pipe to the waste liquid cell via the first liquid discharge pipe; or
the blood sample analyzer further comprises a waste liquid cell, and the analysis and control device is further configured to control the first power source to drive discharge of the cleaning liquid after cleaning of the ESR test pipe to the waste liquid cell via the sample aspiration pipe and the sample collecting needle.

6. The blood sample analyzer of claim 2, **characterized in that** the blood sample analyzer further comprises a cleaning cell and a valve group, and the first cleaning liquid supply device comprises a first liquid storage container configured to store the alkaline cleaning liquid;
the first power source is switchable to be connected to the sample aspiration pipe, the first liquid storage container and the cleaning cell by means of the valve group; and
the analysis and control device is further configured to:
control the valve group to switch to a state in which the first power source is in conduction with the first liquid storage container, and control the first power source to aspirate the alkaline cleaning liquid from the first liquid storage container;
control the valve group to switch to a state in which the first power source is in conduction with the cleaning cell, and control the first power source to deliver the aspirated alkaline cleaning liquid to the cleaning cell; and
control the valve group to switch to a state in which the first power source is in conduction with the sample aspiration pipe, and control the first power source to drive the sample collecting needle to aspirate the alkaline cleaning liquid from the cleaning cell and deliver the alkaline cleaning liquid to the ESR test pipe, so as to clean the ESR test pipe.

7. The blood sample analyzer of claim 1, **characterized in that** the blood sample analyzer further comprises a temporary storage cell;
the sample collecting and dispensing device comprises a sample collecting needle, a sample aspiration pipe, a first power source and a third power source, the sample aspiration pipe being connected to the sample collecting needle and the first power source respectively, and the ESR test pipe being connected to the temporary storage cell and the third power source respectively; and
the analysis and control device is further configured to control the first power source to drive the sample collecting needle to aspirate the blood sample from the sample container, control the first power source to drive discharge of at least part of the blood sample aspirated by the sample collecting needle to the temporary storage cell, and control the third power source to drive delivery of at least part of the blood sample in the temporary storage cell to the ESR test pipe.

8. The blood sample analyzer of claim 7, **characterized in that** the first cleaning liquid supply device comprises a first liquid storage container and a second power source, the first liquid storage container being configured to store the alkaline cleaning liquid, and the second power source being connected to the first liquid storage container and the temporary storage cell respectively; and
the analysis and control device is further configured to control, after completion of the ESR test in the ESR test pipe, the second power source to drive delivery of the alkaline cleaning liquid in the first liquid storage container to the temporary storage cell, and control the third power source to drive delivery of the alkaline cleaning liquid in the temporary storage cell to the ESR test pipe, so as to clean the temporary storage cell and the ESR test pipe;
or,
the first cleaning liquid supply device comprises a first liquid storage container configured to store the alkaline cleaning liquid;
the blood sample analyzer further comprises a second control valve, through which the third power source is switchable to be connected to the ESR test pipe and the first liquid storage container; and
the analysis and control device is further configured to, after completion of the ESR test in the ESR test pipe, control the second control valve to switch to a state in which the third power source is in conduction with the first liquid storage container, then control the third power source to drive aspiration of the alkaline cleaning liquid from the first liquid storage container, then control the second control valve to switch to a state in which the third power source is in conduction with the ESR test pipe, and control the third power source to drive delivery of the alkaline cleaning liquid to the ESR test pipe and the temporary storage cell, so as to clean the ESR test pipe and the temporary storage cell.

9. The blood sample analyzer of claim 1, **characterized in that** the blood sample analyzer further comprises a temporary storage cell and a third control valve;
the sample collecting and dispensing device comprises a sample collecting needle, a sample aspiration pipe and a first power source, the sample aspiration pipe being connected to the sample collecting needle and the first power source respectively, and the ESR test pipe being connected to the temporary storage cell and the first power source respectively;
the first power source is switchable to be connected to the sample aspiration pipe and the ESR test pipe by means of the third control valve; and
the analysis and control device is further configured to control the third control valve to switch to a state in which the first power source is in conduction with the sample aspiration pipe, control the first power source to drive the sample collecting needle to aspirate the blood sample from the sample container, control the first power source to drive discharge of at least part of the blood sample aspirated by the sample collecting needle to the temporary storage cell, control the third control valve to switch to a state in which the first power source is in conduction with the ESR test pipe, and control the first power source to drive delivery of at least part of the blood sample in the temporary storage cell to the ESR test pipe.

10. The blood sample analyzer of claim 1, **characterized in that** the blood sample analyzer further comprises a blood routine test device and a second cleaning liquid supply device that is configured to supply a neutral cleaning liquid;
the blood routine test device comprises a test cell and a blood routine test component, the sample collecting and dispensing device is further configured to dispense at least part of the aspirated blood sample to the test cell or to a reaction cell that is connected to the test cell by means of a pipe, and the blood routine test component is configured to perform a blood routine test on the blood sample in the test cell, the blood routine test comprising at least one of differential leukocyte count, reticulocyte count, hemoglobin measurement, erythrocyte count and platelet count; and
the analysis and control device is further configured to control delivery of the neutral cleaning liquid supplied by the second cleaning liquid supply device to the test cell after completion of the blood routine test in the test cell, so as to clean the test cell.

11. The blood sample analyzer of claim 10, **characterized in that** the sample collecting and dispensing device comprises a sample collecting needle, a sample aspiration pipe and a first power source;
the ESR test pipe is connected to the sample aspiration pipe, or a portion of the sample aspiration pipe is used as the ESR test pipe;
the sample aspiration pipe is connected to the sample collecting needle, the first power source and the test cell respectively, the ESR test pipe being located between the sample collecting needle and the test cell, or the sample aspiration pipe is connected to the sample collecting needle, the first power source and the reaction cell respectively, the ESR test pipe being located between the sample collecting needle and the reaction cell; and
the analysis and control device is configured to control the first power source to deliver at least part of the blood sample aspirated by the sample collecting needle to the ESR test pipe to perform the ESR test, and then control, after completion of the ESR test, the first power source to deliver the blood sample in the ESR test pipe to the test cell or the reaction cell;
or,
the sample collecting and dispensing device comprises a sample collecting needle, a sample aspiration pipe and a first power source;
the ESR test pipe is connected to the sample aspiration pipe, or a portion of the sample aspiration pipe is used as the ESR test pipe;
the sample aspiration pipe is connected to the sample collecting needle and the first power source respectively; and
the analysis and control device is configured to control the first power source to dispense at least part of the blood sample aspirated by the sample collecting needle to the test cell or the reaction cell via the sample collecting needle, and then control the first power source to deliver at least part of the blood sample aspirated by the sample collecting needle to the ESR test pipe; or control the first power source to deliver at least part of the blood sample aspirated by the sample collecting needle to the ESR test pipe, and then control the first power source to dispense at least part of the blood sample aspirated by the sample collecting needle to the test cell or the reaction cell via the sample collecting needle.

12. The blood sample analyzer of any one of claims 1 to 11, **characterized in that** the blood sample analyzer further comprises a second cleaning liquid supply device configured to supply a neutral cleaning liquid; and
the analysis and control device is further configured to: after completion of the ESR test in the ESR test pipe, deliver the alkaline cleaning liquid supplied by the first cleaning liquid supply device to the ESR test pipe to clean the ESR test pipe, and then deliver the neutral cleaning liquid supplied by the second cleaning liquid supply device to the ESR test pipe to clean the ESR test pipe.

13. The blood sample analyzer of claim 12, **characterized in that**, after completion of one ESR test, the ESR test pipe is sequentially cleaned with the alkaline cleaning liquid and the neutral cleaning liquid, so as to ensure that the ESR test pipe does not affect measurement of a level of erythrocyte aggregation of a next blood sample.

14. The blood sample analyzer of any one of claims 1 to 13, **characterized in that** the alkaline cleaning liquid contains at least one surfactant.

15. A method for controlling a blood sample analyzer, **characterized in that** the method comprises:
controlling a sample collecting and dispensing device to aspirate a blood sample from a sample container;
controlling the sample collecting and dispensing device to dispense at least part of the aspirated blood sample to an ESR test pipe;
controlling a photoelectric measurement component to measure a level of erythrocyte aggregation of the blood sample in the ESR test pipe;
analyzing measurement data related to the level of erythrocyte aggregation measured by the photoelectric measurement component to obtain an erythrocyte sedimentation rate of the blood sample; and
controlling delivery of an alkaline cleaning liquid supplied by a first cleaning liquid supply device to the ESR test pipe, so as to clean the ESR test pipe.

16. The method for controlling a blood sample analyzer of claim 15, **characterized in that** the control method further comprises, after the ESR test pipe is cleaned with the alkaline cleaning liquid, controlling delivery of a neutral cleaning liquid supplied by a second cleaning liquid supply device to the ESR test pipe, so as to clean the ESR test pipe.
